# EUROPEAN PATENT SPECIFICATION

(11) **EP 2 574 296 B1**
(45) Date of publication and mention of the grant of the patent: **02.03.2016**
(21) Application number: 11183202.8
(22) Date of filing: 28.09.2011
(51) Int. Cl.: A61B 17/70

(54) **BONE ANCHORING ASSEMBLY**
KNOCHENVERANKERUNGSANORDNUNG
ENSEMBLE D'ANCRAGE D'OS

(43) Date of publication of application: 03.04.2013
(73) Proprietor: Biedermann Technologies GmbH & Co. KG, 78166 Donaueschingen (DE)
(72) Inventor: Biedermann, Lutz, 78048 VS-Villingen (DE); Pohl, Gerhard, 78112 St. Georgen (DE); Matthis, Wilfried, 79367 Weisweil (DE)
(74) Representative: Prüfer & Partner mbB Patentanwälte · Rechtsanwälte

(56) References cited:
- EP-A1- 2 116 205
- EP-A1- 2 135 574
- EP-A1- 2 160 988
- US-A1- 2005 277 920
- US-A1- 2006 083 603
- US-A1- 2006 122 599

## Description

The invention relates to a bone anchoring assembly for dynamic stabilization comprising a bone anchoring element having a head and a shank to be anchored in a bone or a vertebra, and a receiving part for receiving a rod, the rod having a flexible section which is made at least partly of a polymer material. The receiving part comprises a first channel with an approximately U-shaped cross-section with two free legs. Furthermore, a locking element is provided directly cooperating with the legs to secure the rod in the first channel, wherein a first pin-shaped projection is provided at the locking element which comes into contact with the flexible section of the rod when tightening the locking element in such a way that in the tightened state the integral structure of the rod is not violated.

A bone anchoring assembly with a flexible rod made of an elastomer material is known, for example, from EP 1 759 646 A1. The rod is held in the receiving part by means of a closure cap and a filling piece which presses onto the rod when the closure cap is screwed onto the receiving part. The surface of the filling piece and the bottom of the receiving part comprises conical pins which press onto the rod and create an indirect form-fit connection which contributes to the frictional connection so as to hold the rod in place. The indirect form-fit connection is achieved by a local elastic or plastic deformation of the material of the rod. The bone anchoring element is of the type of a monoaxial screw, i.e. the receiving part and the shank are not pivotably connected.

A bone anchoring assembly with a flexible rod is further known from EP 1 795 134 A1 which describes a polyaxial bone anchoring element. The receiving part and the shank are pivotably connected and a pressure element is provided to lock the angular position of the shank relative to the receiving part. The surface of the filling piece and that of the pressure element which contacts the rod have rib-like projections which press onto the flexible rod and provide a form-fit contribution to the fixation of the rod in the receiving part.

From EP 1 900 334 A1 a bone anchoring assembly of the above mentioned type is known, which comprises a single part closure element instead of a closure element with a filling piece. The single part closure element is an inner screw to be screwed between the legs of the receiving part, which has an annular projection on its lower side which presses onto the flexible rod. On the bottom of the channel of the receiving part rib-like projections are provided.

The bone anchoring assemblies mentioned above which use the flexible rod comprise an engagement structure to clamp the rod which has sharp edges and/or which has teeth or ribs which are arranged exactly on opposite sides of the rod in order to provide a safe locking.

With such engagement structures there is a risk of weakening the rod, if the rod diameter is small. Therefore, the known assemblies are mainly used with rods having a relatively large diameter, for example a diameter of approximately 9 mm or larger. However, there is a need for the use of bone anchoring assemblies of the type using a flexible rod which are small in size, in particular, when the implant is to be placed at a location which is exposed and not covered enough by muscles, ligaments or other soft tissue.

EP 2 135 574 A1 discloses a bone anchoring assembly for dynamic stabilization comprising a bone anchoring element with a shank to be anchored in a bone or a vertebra and a receiving part for receiving a rod, wherein the rod is at least partly flexible, the flexible section being made of a polymer material. The rod connects at least two bone anchoring elements. The bone anchoring element comprises a two-piece locking device for allowing a clamping of the rod in two steps.

EP 2 160 988 A1 similarly discloses a rod-shaped implant and a bone anchoring element for stabilizing the spinal column. A two-piece locking device is provided to fixate the rod wherein an inner screw has a pin-shaped projection, and to lock the head of a bone screw element independently from fixating the rod.

US 2005/0277920 A1 discloses a spinal anchoring device wherein a spinal fixation element is locked within a head of an anchoring element by means of an engagement mechanism formed as a single piece. The engagement mechanism comprises a threaded portion and a distal portion formed as a spike or pin, which is configured to fully penetrate through the spinal fixation-element.

US 2006/0122599 A1 similarly discloses a head of a bone anchor in which an elongate member is fixated by means of a coupling member threaded into a recess or passage formed in the head. The coupling member is of a single piece and has a plus body with a thread form, and a penetreating element at its distal end. The penetrating element extends completely through elongate member.

EP 2 116 205 A1 discloses a pedicle screw with a receiving part in which a U-shaped recess is formed, wherein a fixation screw may be threaded in the recess to fixate rod-shaped implant. The fixation screw has a ring-shaped projection at its bottom face, which presses onto rodshaped implant thereby determine the surface to establish a form-fit connection

US 2006/0083603 A1 discloses a spinal fixation anchor having break-off extensions extending from spaced apart arms, which join at a U-shaped rod seat adjacent a shank. The break-off extensions may be removed after advancement of a single-piece closure to fix the rod.

It is an object of the invention to provide an improved bone anchoring assembly for dynamic stabilization using a flexible rod which is smaller in size compared to the known bone anchoring assemblies while providing the same degree of safe fixation of the rod as the known bone anchoring assemblies and which allows an improved handling.

The object is solved by a bone anchoring assembly according to claim 1. Further developments are given in the dependent claims.

The bone anchoring assembly has the advantage that it can be used, for example, with flexible elastomer rods, which have diameters below 9 mm. When using a small diameter rod in particular the receiving part, can also be down-sized. Therefore, a low profile implant is provided which has the advantage that the irritation of surrounding body material is small. By the one-part locking element the bone anchoring assembly has only few parts. The one-step clamping of the rod is easy, safe and effective, wherein only one tool is needed for screwing-in the single locking element. Therefore, an easy and comfortable handling of the bone anchoring assembly is possible.

When the surgeon would like to connect three or more bone anchoring devices in a row via a rod, by the tube-shaped extension the rod can be easily pressed down via the inner screw even if the bone anchoring devices are mounted on different levels in the body. The rod is still located between the legs of the receiving part or the tube-shaped extension.

The pins that contribute to clamp the rod have a rounded tip. Hence, the integrity of the surface of the rod is not violated, since the pins do not scratch the structure.

Mechanical stops are provided preventing a penetration of the clamping pins into the surface of the rod due to limitation of the pressure force.

Further features and advantages will become apparent from the description of embodiments by means of the accompanying drawings.

In the drawings:
- Fig. 1a: shows a perspective exploded view of a bone anchoring assembly.
- Fig. 1b: shows a perspective view of the bone anchoring assembly according to Fig. 1a in an assembled state in a first configuration.
- Fig. 1c: shows a perspective view of the bone anchoring assembly in an assembled state in a third configuration.
- Fig. 2a: shows a perspective view of the bone anchoring assembly in the first configuration.
- Fig. 2b: shows a perspective view of the bone anchoring assembly in a second configuration.
- Fig. 2c: shows a perspective view of the bone anchoring assembly in the third configuration.
- Fig. 2d: shows a perspective view of the bone anchoring assembly in a fourth configuration.
- Fig. 3a: shows a first cross-sectional view of the bone anchoring assembly in the first configuration.
- Fig. 3b: shows the first cross-sectional view of the bone anchoring assembly in the second configuration.
- Fig. 3c: shows the first cross-sectional view of the bone anchoring assembly in the third configuration.
- Fig. 3d: shows the first cross-sectional view of the bone anchoring assembly in the fourth configuration.
- Fig. 4a: shows a second cross-sectional view of the bone anchoring assembly in the first configuration.
- Fig. 4b: shows the second cross-sectional view of the bone anchoring assembly in the second configuration.
- Fig. 4c: shows the second cross-sectional view of the bone anchoring assembly in the third configuration.
- Fig. 4d: shows the second cross-sectional view of the bone anchoring assembly in the fourth configuration.

A bone anchoring assembly comprises one or more (not shown) bone anchoring devices 1 and a rod 8, wherein the bone anchoring devices 1 can be interconnected via the rod 8.

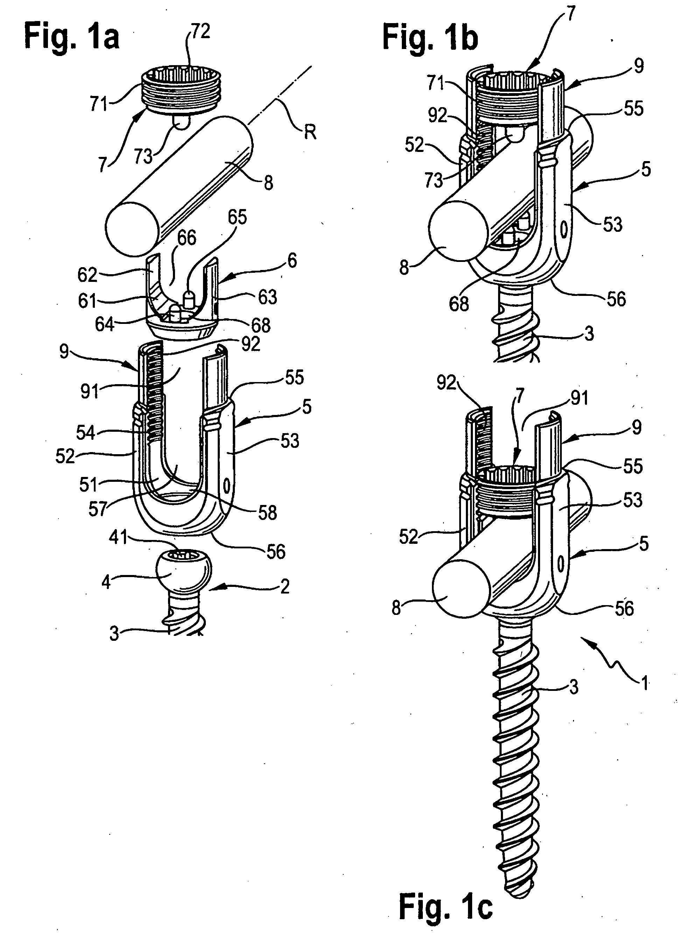

As shown in Figs. 1a to 4d, the bone anchoring device 1 comprises a bone anchoring element 2 in the form of a polyaxial bone screw having a shank 3 with a bone thread and a tip at one end and a head 4 having an engagement structure 41 at the opposite end. The bone anchoring device 1 furthermore comprises a receiving part 5, a pressure element 6, a locking element 7 and a tube-shaped extension 9 which is integrally formed with the receiving part 5.

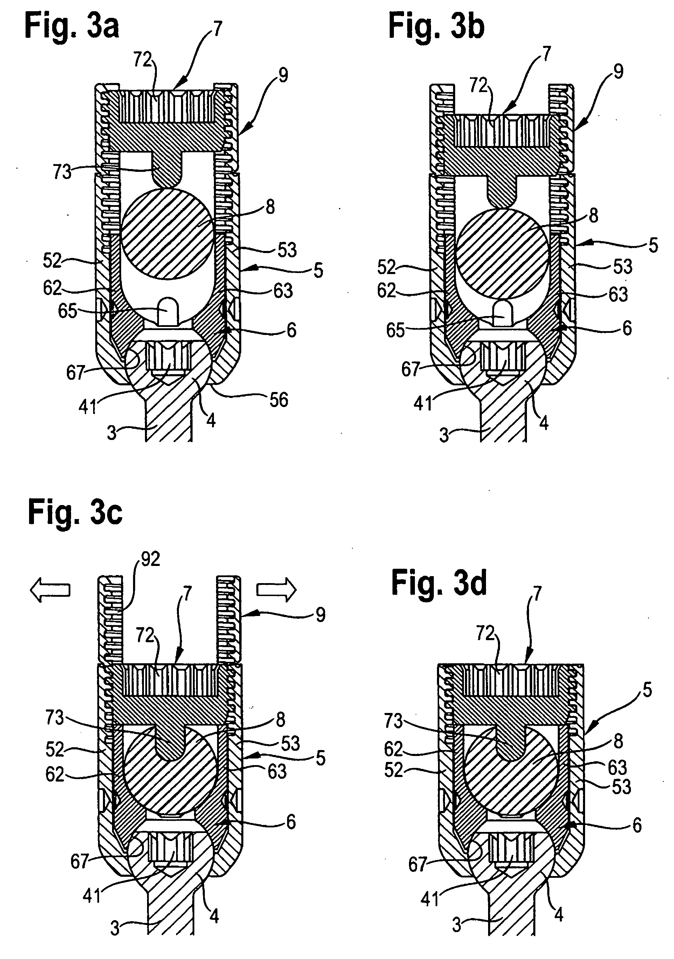

The receiving part 5 is substantially cylindrical and comprises a first end 55, a second end 56 and a coaxial bore 57 extending from the first end 55 to the second end 56 and tapering in an area near the second end 56 such that, as shown in Fig. 3a, the head 4 of the bone anchoring element 2 is pivotably held in the receiving part 5 at the second end 56. Furthermore, the receiving part 5 comprises a U-shaped recess 51 extending from the first end 55 in the direction of the second end 56. By means of the U-shaped recess 51 two free legs 52, 53 are formed which comprise an internal thread 54, wherein a channel 58 is formed.

The pressure element 6 is substantially cylindrical and dimensioned such that it can be moved within the bore 57 of the receiving part 5. The pressure element 6 has a coaxial bore 66 extending through the pressure element 6 and allowing to guide a screwing-in tool therethrough for screwing-in the bone anchoring element 2 into a bone. The pressure element 6 further comprises a spherical recess 67, which is adapted to receive the spherical head 4 of the bone anchoring element 2. Further, the pressure element 6 comprises a substantially U-shaped recess 61 extending from its free end in the direction of the spherical recess 67. By means of the U-shaped recess 61 two free legs 62, 63 are formed which form the lateral walls of a channel 68 for receiving the rod 8. On the bottom of the channel 68 two pin-shaped projections 64, 65 or pins 64, 65 are provided which are located preferably at both ends of the channel 68. The pins 64, 65 are substantially cylindrical and their free end is rounded, preferably semi-spherical. More specifically, the pins 64, 65 are longitudinal rod-shaped pins with a rounded free end. However, they can have any shape as long as the uppermost portion is rounded as described. The uppermost portions of the pins 64, 65 are located on a line, which is parallel to the longitudinal axis R of the rod 8.

As shown in particular in Fig. 3c the pressure element 6 is sized in such a way that the legs 62, 63 of the pressure element 6 extend slightly above the surface of the rod 8 when the rod 8 is inserted into the channel 68 and the pins 64, 65 are immersed into the surface of the rod 8. The upper edge of the legs 62, 63 of the pressure element 6 form a stop for the main body of the locking element 7.

The locking element 7 is a single part locking element and can be formed as an inner screw and comprises an outer thread 71, an engagement structure 72 for engagement with a tool and a coaxial pin 73 for pressing onto the rod 8. The pin 73 is preferably cylindrical with a semi-spherical free end, more specifically, the pin 73 is a longitudinal rod-shaped pin with a rounded free end and corresponds in its dimension substantially to the dimensions of the pins 64, 65 of the pressure element 6.

The bone anchoring element 2, the receiving part 5, the pressure element 6, the locking element 7 and also the tube-shaped extension 9 can be made of a biocompatible material, such as, for example, titanium or stainless steel or another biocompatible material, for example PEEK.

The rod 8 is made at least in part of a flexible biocompatible material, preferably of a plastic material and in particular of an elastomer material. Such a material can be based on, for example, polycarbonate-polyurethane or polycarbonate-urethane (PCU). However, other materials are also applicable, for example styrene-block-isobutylene-block-styrene (SIBS) and other elastomers. The rod 8 needs not to be made totally of one single material, but can comprise several materials and inner structures and/or sections with different flexibility and/or rigidity. The flexible section is the section which is to be clamped in the receiving part 5. The diameter of the rod 8 can be any of the usual smaller diameters of rods for stabilization of the spine, in particular diameters from 4.5 mm to 9 mm.

The coaxial tube-shaped extension 9 has a slot 91 for inserting the rod 8 and an internal thread 92 which cooperates with the inner thread 54 of the receiving part 5. The extension 9 is used for minimally invasive surgery and can be broken away after tightening the locking element 7, preferably manually. For breaking away the tube-shaped extension 9 in an easy way, a predetermined breaking point, i.e. a material weakness formed by a groove between the receiving part 5 and the tube-shaped extension 9 for example can be provided.

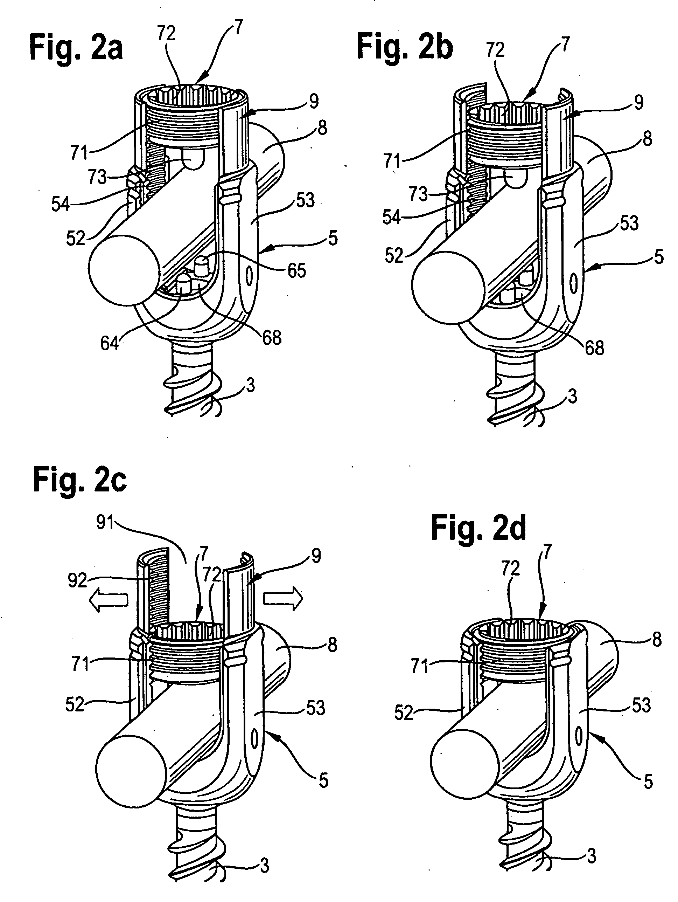

In use, as can be seen from Figs. 2a to 4d, firstly at least two bone anchoring devices 1 are screwed into adjacent vertebrae, for example into the pedicles. At least one of the bone anchoring devices 1 is formed according to the invention. Thereafter, the rod 8 is inserted into the receiving part 5 and is fixed. The procedure of fixation is now explained with reference to Figs. 2a to 4d. As shown in Figs. 2a, 3a, 4a, in a first configuration the locking element 7 is screwed into the tube-shaped extension 9. The rod 8 is inserted into the channel 68 until it rests onto the pins 64, 65 of the pressure element 6 or on the pin 73 of the locking element 7. As can be seen from Figs. 2b, 2c, 3b, 3c, 4b, 4c, when the locking element 7 is screwed-in completely between the legs of the tube-shaped extension 9 and then between the legs 52, 53 of the receiving part 5, the pins 64, 65 are pressing into the surface of the rod 8 until they are fully immersed in the surface of the rod 8 due to the local flow of the material of the rod 8 which leads to a local elastic and/or plastic deformation of the rod 8 The engagement of the pins 64, 65 with the rod 8 is such that the integral structure of the rod 8 is not violated.

The pin 73 of the locking element 7 is also fully immersed in the surface of the rod 8 due to the local flow of the material of the rod 8 which leads to a local elastic and/or plastic deformation of the rod 8.

Due to the mechanical stop by the upper edge of the pressure element 6, the movement of the locking element 7 is restricted.

After screwing in the full immersion of the pins 64, 65, 73 the tube-shaped extension 9 is broken away via the predetermined breaking point after tightening the locking element 7 as indicated in Figs. 2c, 3c by the arrows.

The dimension of the pins 64, 65, 73, in particular their height, the diameter and the radius of the free end portion is designed such that under a given pressure force which is limited by the stop described above, the pins 64, 65, 73do not violate the integral structure of the rod 8.

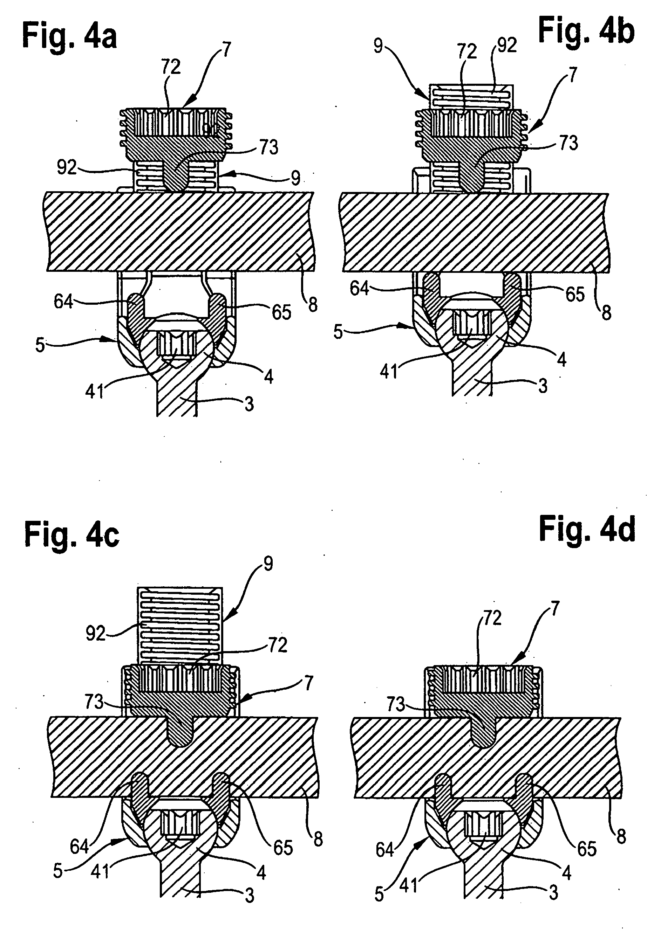

As can be seen in particular in Figs. 4c, 4d, the arrangement of the pin fixation seen in the direction perpendicular to the longitudinal axis of the rod 8 is a three-point fixation which is particularly safe. That means, there is no clamping on locations which are exactly on opposite sides of the rod 8 which may cause the danger of violating the integral structure of the rod 8 at the clamping site.

Several modifications are conceivable. For example, the number of pins in the bottom of the channel of the pressure element or also the number of pins of the locking element may vary. In some cases more than two pins might be of advantage referring to the pressure element. The shape of the pins can also vary. However, the height of the pins and the radius of the uppermost rounded portion must be designed such that there is no violation of the integral structure of the rod, while simultaneously providing safe fixation.

All other kinds of polyaxial bone anchoring assemblies known may be conceivable which can be modified so as to have the pins described above. For example, a polyaxial screw, where the bone anchor is inserted from below, a so-called bottom-loader, may be also used.

## Claims

1. Bone anchoring assembly for dynamic stabilization, comprising:
a bone anchoring element (2) having a head (4) and a shank (3) to be anchored in a bone or a vertebra, and a receiving part (5) for receiving a rod (8),
a rod (8) having a flexible section which is at least partly made of a polymer material, wherein
the receiving part (5) comprising a first channel (58) with an approximately U-shaped cross-section with two free legs (52, 53),
a single-part locking element (7) directly cooperating with the legs (52, 53) to secure the rod (8) in the first channel (58), wherein
a first pin-shaped projection (73) is provided at the locking element (7) which comes into contact with the flexible section of the rod (8) when tightening the locking element (7),
said first pin-shaped projection (73) having a height and a semi-spherically rounded free end portion including a diameter and radius in such a way that in the tightened state the integral structure of the rod (8) is not violated
wherein a tube-shaped extension (9) is provided which is integrally formed with the receiving part (5) and having a length which allows the locking element (7) to cooperate with the legs (52, 53) until the rod (8) inserted into the first channel (58) rests on the pin-shaped projection (73).

2. The bone anchoring assembly according to claim 1, wherein the bone anchoring element (2) and the receiving part (5) are pivotably connected.

3. The bone anchoring assembly according to claim 1 or 2, wherein a pressure element (6) is provided which exerts pressure onto the head (4) of the bone anchoring element (2) to lock the angular position of the bone anchoring element (2) relative to the receiving part (5).

4. The bone anchoring assembly according to claim 3, wherein the pressure element (6) comprises a second channel (68) with an approximately U-shaped cross-section.

5. The bone anchoring assembly according to claim 4, wherein at least one second pin-shaped projection (64, 65) is provided in the second channel (68) which comes into contact with the rod (8) when tightening the locking element (7).

6. The bone anchoring assembly according to claim 5, wherein the first and second pin-shaped projection (73, 64, 65) is a longitudinal rod-shaped pin and the surface of the first and second pin-shaped projection (73, 64, 65) which contacts the rod (8), is rounded.

7. The bone anchoring assembly according to claim 5 or 6, wherein a plurality of second pin-shaped projections (64, 65) is arranged on the bottom of the second channel (68).

8. The bone anchoring assembly according to claim 7, wherein the second pin-shaped projections (64, 65) are arranged along a line, which is parallel to the longitudinal axis (R) of the rod (8).

9. The bone anchoring assembly according to one of the claims 1 to 8, wherein the locking element (7) is a screw with an external thread (71) engaging the legs (52, 53) of the receiving part (5).

10. The bone anchoring assembly according to one of the claims 7 to 9, wherein the second pin-shaped projections (64, 65) are provided at both outer ends of the second channel (68).

11. The bone anchoring assembly according to one of the claims 1 to 10, wherein a stop is provided which limits the insertion of the locking element (7) into the receiving part (5).

12. The bone anchoring assembly according to one of the claims 7 to 11, wherein two second pin-shaped projection (64, 65) are provided in an axial offset to the first pin-shaped projection (73) in such a manner that a three-point fixation is achieved.

13. The bone anchoring assembly according to one of claims 1 to 12, wherein the tube-shaped extension (9) can be broken away from the receiving part (5), preferably via a predetermined breaking point.

14. The bone anchoring assembly according to one of the claims 1 to 13, wherein the rod (8) is made at least partly of an elastomer material.

## Patentansprüche

1. Knochenverankerungsanordnung zur dynamischen Stabilisierung, umfassend:
ein Knochenverankerungselement (2) mit einem Kopf (4) und einem Schaft (3) zur Verankerung in einem Knochen oder einem Wirbel, und einem Aufnahmeteil (5) zur Aufnahme eines Stabs (8),
einen Stab (8) mit einem flexiblen Abschnitt, der zumindest teilweise aus einem Polymermaterial gefertigt ist, worin
das Aufnahmeteil (5) einen ersten Kanal (58) mit einem ungefähr U-förmigen Querschnitt mit zwei freien Schenkeln (52, 53) umfasst,
ein einteiliges Verriegelungselement (7), das direkt mit den Schenkeln (52, 53) zur Fixierung des Stabs (8) in dem ersten Kanal (58) zusammenarbeitet, worin
ein erster stiftförmiger Vorsprung (73) an dem Verriegelungselement (7) vorgesehen ist, der mit dem flexiblen Abschnitt des Stabs (8) beim Anziehen des Verriegelungselements (7) in Berührung kommt,
wobei der erste stiftförmige Vorsprung (73) eine Höhe und einen halbkugelförmig abgerundeten freien Endabschnitt mit einem Durchmesser und einem Radius aufweist, so dass im angezogenen Zustand die integrale Struktur des Stabs (8) nicht verletzt wird,
worin eine röhrenförmige Verlängerung (9) bereitgestellt ist, die einstückig mit dem Aufnahmeteil (5) geformt ist und eine Länge aufweist, die es dem Verriegelungselement (7) gestattet, mit den Schenkeln (52, 53) zusammenzuarbeiten, bis der in den ersten Kanal (58) eingeführte Stab (8) auf dem stiftförmigen Vorsprung (73) ruht.

2. Knochenverankerungsanordnung nach Anspruch 1, worin das Knochenverankerungselement (2) und das Aufnahmeteil (5) schwenkbar verbunden sind.

3. Knochenverankerungsanordnung nach Anspruch 1 oder 2, worin ein Druckelement (6) bereitgestellt ist, das Druck auf den Kopf (4) des Verankerungselements (2) zur Verriegelung der Winkelstellung des Knochenverankerungselements (2) relativ zu dem Aufnahmeteil (5) ausübt.

4. Knochenverankerungsanordnung nach Anspruch 3, worin das Druckelement (6) einen zweiten Kanal (68) mit einem ungefähr U-förmigen Querschnitt umfasst.

5. Knochenverankerungsanordnung nach Anspruch 4, worin zumindest ein zweiter stiftförmiger Vorsprung (64, 65) in dem zweiten Kanal (68) bereitgestellt ist, der mit dem Stab (8) beim Anziehen des Verriegelungselements (7) in Berührung kommt.

6. Knochenverankerungsanordnung nach Anspruch 5, worin der erste und der zweite stiftförmige Vorsprung (73, 64, 65) ein langgestreckter stabförmiger Stift ist und die Oberfläche des ersten und zweiten stiftförmigen Vorsprungs (73, 64, 65), die den Stab (8) berührt, abgerundet ist.

7. Knochenverankerungsanordnung nach Anspruch 5 oder 6, worin eine Vielzahl von zweiten stiftförmigen Vorsprüngen (64, 65) auf dem Boden des zweiten Kanals (68) angeordnet ist.

8. Knochenverankerungsanordnung nach Anspruch 7, worin die zweiten stiftförmigen Vorsprünge (64, 65) entlang einer Linie angeordnet sind, die parallel zu der Längsachse (R) des Stabs (8) ist.

9. Knochenverankerungsanordnung nach einem der Ansprüche 1 bis 8, worin das Verriegelungselement (7) eine Schraube mit einem Außengewinde (71) in Eingriff mit den Schenkeln (52, 53) des Aufnahmeteils (5) ist.

10. Knochenverankerungsanordnung nach einem der Ansprüche 7 bis 9, worin die zweiten stiftförmigen Vorsprünge (64, 65) an beiden Außenenden des zweiten Kanals (68) bereitgestellt sind.

11. Knochenverankerungsanordnung nach einem der Ansprüche 1 bis 10, worin ein Anschlag bereitgestellt ist, der die Einführung des Verriegelungselements (7) in den Aufnahmeteil (5) begrenzt.

12. Knochenverankerungsanordnung nach einem der Ansprüche 7 bis 11, worin zwei zweite stiftförmige Vorsprünge (64, 65) in einem axialen Versatz zu dem ersten stiftförmigen Vorsprung (73) auf eine solche Weise bereitgestellt sind, dass eine Dreipunkt-Fixierung erzielt wird.

13. Knochenverankerungsanordnung nach einem der Ansprüche 1 bis 12, worin die röhrenförmige Verlängerung (9) von dem Aufnahmeteil (5) weggebrochen werden kann, vorzugsweise über eine vorbestimmte Bruchstelle.

14. Knochenverankerungsanordnung nach einem der Ansprüche 1 bis 13, worin der Stab (8) zumindest teilweise aus einem Elastomermaterial gefertigt ist.

## Revendications

1. Ensemble ancrage osseux pour une stabilisation dynamique, comprenant :
un élément d'ancrage osseux (2) comportant une tête (4) et une tige (3) à ancrer dans un os ou une vertèbre et une pièce réceptrice (5) pour recevoir une tige (8),
une tige (8) ayant une section flexible qui est au moins en partie faite d'une matière polymère, dans lequel
la pièce réceptrice (5) comprend un premier canal (58) ayant une section transversale à peu près en forme d'U avec deux pattes libres (52, 53),
un élément de verrouillage (7) d'une seule pièce coopérant directement avec les pattes (52, 53) pour fixer solidement la tige (8) dans le premier profilé en U (58), dans lequel
une première saillie (73) en forme de clavette est disposée sur l'élément de verrouillage (7) et vient en contact avec la section flexible de la tige (8) quand on serre l'élément de verrouillage (7),
ladite première saillie (73) en forme de clavette ayant une hauteur et une partie d'extrémité libre arrondie en demi-sphère comprenant un diamètre et un rayon de telle façon que, dans l'état serré, la structure intégrale de la tige (8) n'est pas mise à mal,
dans lequel est disposé un prolongement (9) en forme de tube qui fait partie intégrante de la pièce réceptrice (5) et a une longueur qui permet à l'élément de verrouillage (7) de coopérer avec les pattes (52, 53) jusqu'à ce que la tige (8) introduite dans le premier profilé en U (58) s'appuie sur la saillie (73) en forme de clavette.

2. Ensemble ancrage osseux selon la revendication 1, dans lequel l'élément d'ancrage osseux (2) et la pièce réceptrice (5) sont raccordés à pivotement.

3. Ensemble ancrage osseux selon la revendication 1 ou 2, dans lequel est disposé un élément de pression (6) qui exerce une pression sur la tête (4) de l'élément d'ancrage osseux (2) pour verrouiller la position angulaire de l'élément d'ancrage osseux (2) par rapport à la pièce réceptrice (5).

4. Ensemble ancrage osseux selon la revendication 3, dans lequel l'élément de pression (6) comprend un second canal (68) ayant une section transversale à peu près en forme d'U.

5. Ensemble ancrage osseux selon la revendication 4, dans lequel au moins une seconde saillie (64, 65) en forme de clavette est disposée dans le second profilé en U (68) et vient en contact avec la tige (8) quand on serre l'élément de verrouillage (7).

6. Ensemble ancrage osseux selon la revendication 5, dans lequel les première et secondes saillies (73, 64, 65) en forme de clavette sont des clavettes longitudinales en forme de tige et la surface des première et secondes saillies (73, 64, 65) en forme de clavette qui entrent en contact avec la tige (8) est arrondie.

7. Ensemble ancrage osseux selon la revendication 5 ou 6, dans lequel une pluralité de secondes saillies (64, 65) en forme de clavette sont disposées sur le fond du second profilé en U (68).

8. Ensemble ancrage osseux selon la revendication 7, dans lequel les secondes saillies (64, 65) en forme de clavette sont agencées le long d'une ligne qui est parallèle à l'axe longitudinal (R) de la tige (8).

9. Ensemble ancrage osseux selon l'une quelconque des revendications 1 à 8, dans lequel l'élément de verrouillage (7) est une vis avec un filetage extérieur (71) se vissant sur les pattes (52, 53) de la pièce réceptrice (5).

10. Ensemble ancrage osseux selon l'une quelconque des revendications 7 à 9, dans lequel les secondes saillies (64, 65) en forme de clavette sont disposées aux deux extrémités extérieures du second profilé en U (68).

11. Ensemble ancrage osseux selon l'une quelconque des revendications 1 à 10, dans lequel est disposée une butée qui limite l'introduction de l'élément de verrouillage (7) dans la pièce réceptrice (5).

12. Ensemble ancrage osseux selon l'une quelconque des revendications 7 à 11, dans lequel deux secondes saillies (64, 65) en forme de clavette sont disposées avec un décalage axial par rapport à la première saillie (73) en forme de clavette de telle manière qu'on obtient une fixation à trois points.

13. Ensemble ancrage osseux selon l'une quelconque des revendications 1 à 12, dans lequel on peut séparer en le brisant le prolongement (9) en forme de tube de la pièce réceptrice (5), de préférence par le biais d'un point de rupture prédéterminé.

14. Ensemble ancrage osseux selon l'une quelconque des revendications 1 à 13, dans lequel la tige (8) est faite au moins en partie d'une matière élastomère.
